Europäisches Patentamt

European Patent Office

Office européen des brevets

(19)

(11) Numéro de publication : **0 349 469 B1**

## (12) FASCICULE DE BREVET EUROPEEN

(45) Date de publication du fascicule du brevet :
**28.07.93 Bulletin 93/30**

(51) Int. Cl.$^5$ : **A61K 7/48, A61K 35/78**

(21) Numéro de dépôt : **89500073.5**

(22) Date de dépôt : **28.06.89**

(54) **Produit pharmaceutique doté d'activité de régénération de l'épiderme basé sur le principe actif du mimosa tenuiflora et procédé d'obtention correspondant.**

(30) Priorité : **28.06.88 ES 8802029**

(43) Date de publication de la demande :
**03.01.90 Bulletin 90/01**

(45) Mention de la délivrance du brevet :
**28.07.93 Bulletin 93/30**

(84) Etats contractants désignés :
**AT BE CH DE GB GR IT LI LU NL SE**

(56) Documents cités :
**EP-A- 0 329 834**
**DE-A- 1 492 129**
**FR-A- 726 015**
**FR-A- 2 510 402**
**FR-A- 2 512 669**
**GB-A- 2 134 389**

(73) Titulaire : **Tellez Perez, Julio**
**Avda. del Taller Retorno 12, No. 9 (Jardin Balbuena)**
**Mexico DF (MX)**
Titulaire : **Dupoy de Guitard, Jacques**
**Isaac Peral No. 46**
**E-28040 Madrid (ES)**

(72) Inventeur : **Tellez Perez, Julio**
**Avda. del Taller Retorno 12, No. 9 (Jardin Balbuena)**
**Mexico DF (MX)**
Inventeur : **Dupoy de Guitard, Jacques**
**Isaac Peral No. 46**
**E-28040 Madrid (ES)**

(74) Mandataire : **Gil Vega, Victor**
**Estébanez Calderon, 3 - 5B**
**E-28020 Madrid (ES)**

## Description

La présente invention concerne un procédé pour l'extraction et l'isolement d'un principe actif contenu dans l'écorce de l'arbre Mimosa tenuiflora, ainsi que les compositions pharmaceutiques contenant le principe actif obtenu directement par ce procédé.

Le principe actif mentionné contient un puissant facteur inducteur de la régénération épidermique et est en particulier indiqué et efficace pour le traitement des brûlures, ulcères, varices, blessures chirurgicales, lésions légères, crevasses, éraflures, écorchures, plaies et eczéma, et est en outre stimulant des follicules pileux.

Etat de la technique

Toutefois, le principe actif de l'invention n'a pas été identifié complètement quant à sa structure.

L'utilisation de l'écorce de la arbre Mimosa tenuiflora, connu sous le nom de TEPESCOHUITE, date du temps du mexique préhispanique jusqu'à nos jours. Ces antécédents remontent à l'utilisation par les Indiens Mayas dans la région sud du pays, lieu d'où provient ladite plante.

Elle a été décrite pour la première fois par D. Francisco Hernández dans son livre "Historia de las Plantas de la Nueva España", pendant le XVIIº siècle, lorsqu'il était employé comme Premier Médecin de Philippe II. Pendant son séjour en Nouvelle Espagne, il se consacra à compiler et à codifier les connaissances de la Botanique Médicinale.

Dans son livre il apparaît sous le nom de TETLATLIZTLI en langue Nahualt et il était largement connu dans la vallée de Mexico par les anciens Mexicains ou Aztèques.

Les natifs de la région de la vallée de Cintalapa, Chiapas et autres régions ont utilisé l'écorce simplement moulue des arbres adultes et sa stérilisation consistait à la griller dans un poêle puis à la saupoudrer sur la brûlure.

En fait, on continue d'utiliser l'écorce de l'arbre Mimosa tenuiflora ou des extraits de cette plante de manière rudimentaire au moyen du saupoudrage sur la blessure, ce qui ne permet pas une utilisation adéquate des propriétés du principe actif ni un dosage correct, raison pour laquelle la profession médicale montre une réticence à utiliser ce moyen de traitement, bien que le document FR-A 726 015 décrit une crème de beauté comprenant des extraits de Mimosa, le document DE-A 1.492 129 décrit un produit de dégraisage des cheveux comprenant le extrait de l'écorce de Mimosa (voir notamment l'exemple 9), le document FR-A 2 512 669 décrit une composition pour traitement capillaire comprenant des composés polyphénoliques tels que les médicaments de tannin qu'on obtient de Mimosa (voir notamment revendications et page 2) et le document GB-A 2 134 389 décrit le traitement cosmétique des ongles à l'aide d'une composition comprenant extraits de Mimosa (voir notamment les revendications et l'exemple 1).

Le nombre de personnes qui souffrent de brûlures augmente dans la majorité des pays industrialisés et en voie de développement, étant donné l'accroissement démographique dans les grandes villes et l'augmentation du taux de natalité.

Une étude statistique réalisée récemment en 1980 aux Etats Unis d'Amérique par l'Organisation Mondiale de la Santé (OMS) et le Département de Santé Publique des Etats Unis montre qu'une personne sur 10 souffrant d'un type quelconque d'accident et entrant dans un centre hospitalier le fait en rapport avec un type quelconque de traumatisme par brûlure. Et parmi ces personnes brûlées entre 200 000 et 300 000 nécessitent une convalescence prolongée de plus d'un mois, plus une phase de rééducation orthopédique qui varie de plusieurs semaines à quelques mois, et parmi ces malades de 10.000 à 12.000 personnes sont mortes pendant l'année 1980.

Pour une personne brûlée sur 50% de la surface du corps, affectée par une lésion qui est surtout du second degré, superficielle ou profonde, dans un pays en voie de développement, le traitement de ces lésions est très coûteux, puisque les coûts quotidiens d'hospitalisation oscillent entre 300 et 1000 $ U.S.

Etant donné la complication des traitements pour l'utilisation de greffes de types distincts et le temps prolongé d'hospitalisation, un calcul prudent varierait approximativement autour de 36 000 $ américains.

D'un autre côté, les traitements utilisés ne sont pas simples étant donné les déficiences des infrastructures de santé dans d'autres pays, où tous les sujets ne bénéficient pas de la même qualité de soins médicaux ; tout ceci étant lié à la prolongation du temps de traitement que requièrent ces malades, le séjour hospitalier étant coûteux.

Ce problème se résoudrait en partie au moyen de l'emploi du produit actif objet de l'invention, qui, en permettant une utilisation maximale des propriétés de l'écorce de Mimosa tenuiflora et au moyen d'une application extrêmement simple, réduirait le séjour à l'hopital du malade, approximativement de 50%.

On voit donc apparaître la nécessité de rechercher et de mettre au point une méthodologie adéquate pour extraire, isoler et caractériser la fraction du principe actif dans l'écorce de Mimosa tenuiflora et une fois isolé des autres composants non actifs, préparer au moyen des excipients et véhicules appropiés des médicaments susceptibles d'une application adéquate et d'un contrôle de leur posologie. C'est là précisement l'un des objets de l'invention.

## DESCRIPTION DE L'INVENTION

### 1.- Objet de l'invention

Un premier objet de l'invention est un procédé d'obtention du principe actif avec propriétés de régéneration épidermique contenu dans l'écorce de Mimosa tenuiflora.

Un second objet de l'invention est constitué par les compositions pharmaceutiques contenant le principe actif obtenu par le procédé faisant l'objet de l'invention.

### 2.- Description des dessins

La figure 1 montre le spectre d'absorption UV-VIS du principe actif extrait de l'écorce de Mimosa tenuiflora.

La figure 2 montre le spectre de résonance infrarouge dudit principe actif.

La figure 3 montre le spectre de résonance magnétique protonique dudit principe actif dans $D_2$ O.

La figure 4 montre le spectre de résonance magnétique C dudit principe actif dans $D_2$ O.

### 3.- Description détaillée de l'invention

Le principe actif avec propriétés de régénération épidermique contenu dans l'écorce de Mimosa tenuiflora et obtenu selon le procédé d'extraction décrit plus loin présente la caractérisation suivante:

a) caractéristiques physiques:

a.1. caractéristiques organoleptiques: ce composé est une poudre de couleur café rougeâtre de saveur amère et inodore.

a.2. point de fusion: on le calcule dans un appareil Fisher-Johns. Le composé actif présente un point de décomposition de 222°C.

a.3. solubilité : cette étude se réalise avec des solvants de polarités distinctes à température ambiante.

| Solvants | mg/ml | Solvants | mg/ml |
|---|---|---|---|
| Ethanol | 98 | Hexane | 0 |
| Diméthylsulfoxyde | 224 | Tétrachlorure de | |
| Méthanol | 175 | carbone | 0 |
| Acétone | 16 | Tétrahydrofuranne | 0 |
| Eau | 0 | Petrolatum | 0 |
| Chloroforme | 0 | Dioxanne | 0 |
| Acétate d'éthyle | 0 | Alcool amylique | 0 |
| Ether éthylique | 0 | Alcool isopropylique | 0 |
| Propanol | 0 | Alcool benzylique | 0 |
| | | Diéthylamine | 0 |

a.4. Essais de pureté : on emploie la technique de chromatographie en couche mince, en utilisant des feuilles chromatographiques Merck DC-Alufolien Kieselgel 60 ce 0,2 mm d'épaisseur en employant les systèmes d'élution suivants :

| Solvants | Relation frontale (R$_f$) |
|---|---|
| a). chloroforme | 0 |
| b). acétate d'éthyle | 0 |
| c). éther de pétrole | 0 |
| d). benzène | 0 |
| e).méthanol-eau-dimétylsulfoxyde 1:1:1 V/V | 0,963 |
| f). éthanol-eau-acide acétique 2:1:1 V/V | 0,817 |
| g). méthanol-diméthylsulfoxyde | 0,910 |
| h). dimétylsulfoxyde | 1.000 |
| i). dioxanne-méthanol-acétate d'éthyle 1:1:1 V/V | 0 |

a.5. contenu en métaux pesés : l'analyse se fait dans un spectrophotométre d'absorption atomique Perkin-Elmer modèle 5000, en obtenant les résultats suivants (en parties par million) :

| Elément | Concentration ppm | Elément | Concentration ppm |
|---|---|---|---|
| Pb | 0 | Mn | 0 |
| Zn | 0 | Cu | 0 |
| Cr | 0 | Hg | 0 |
| Fe | 0 | As | 0 |

a.6. humidité absolue : on calcule au moyen de la technique de poids constant en employant pour ce dosage un gramme de principe actif. L'humidité obtenue est de 10,01%

a.7. résidus de combustion : le résidu obtenu après la combustion d'un gramme de principe actif est de 885 mg de cendres.

a.8. pH en solution : on prépare différentes concentrations de principe actif en solution aqueuse. Les lectures sont faites dans un potentiomètre (Conductronic pH 10), à une température de 25°C. Les résultats obtenus sont donnés ci-dessous :

| Concentration (mg/ml) | pH |
|---|---|
| 100 | 3,94 |
| 177 | 3,11 |
| 316 | 3,09 |
| 562 | 3,08 |
| 1000 | ne se dissout pas |

b) caractéristiques spectrales :

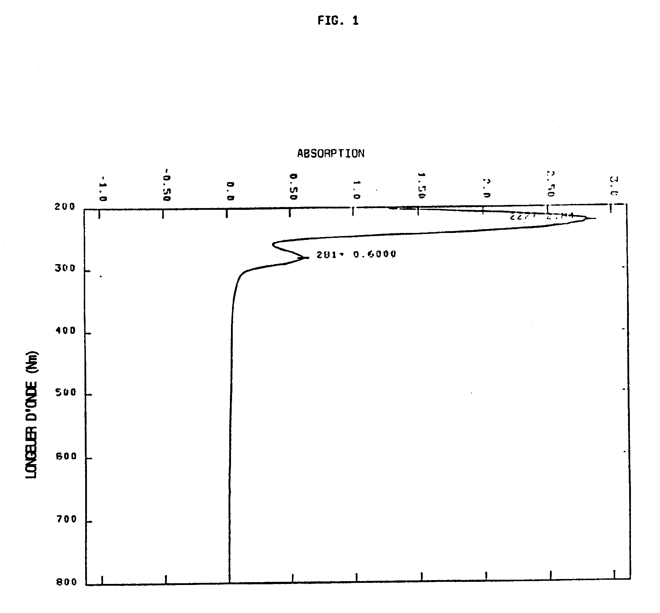

b.1. spectre d'absorption ultraviolette : voir Figure 1
Instrument : Specord UV-VIS
Solvant : éthanol

| λ max (nm) | absorption |
|---|---|
| 222 | 2,840 |
| 281 | 0,600 |

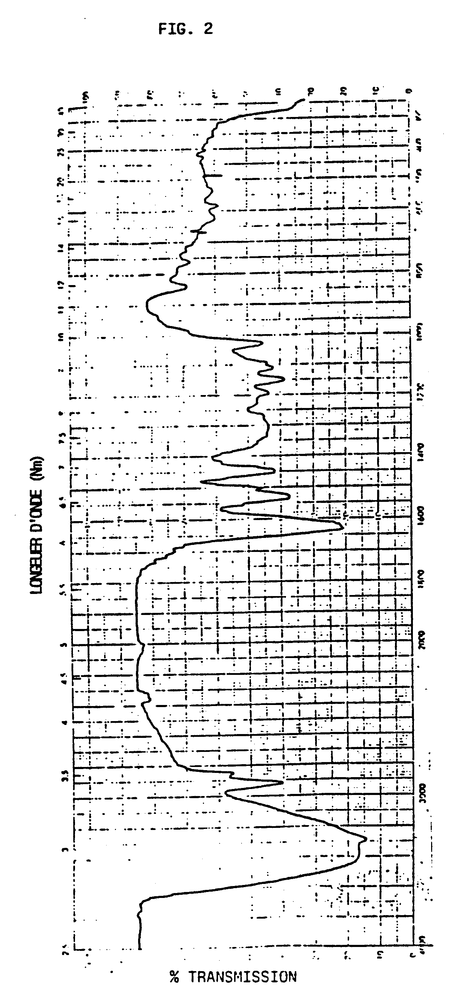

b.2. spectre d'absorption infrarouge : voir Figure 2

Instrument: Specord 1 index chromatographique de retention 75.

En comprimés de bromure de potassium.

Bandes spectroscopiques d'absorption:

3290, 2910, 1630, 1530, 1450, 1190, 1150, 1110, 1020, 840, 760, 620, 580 cm$^{-1}$.

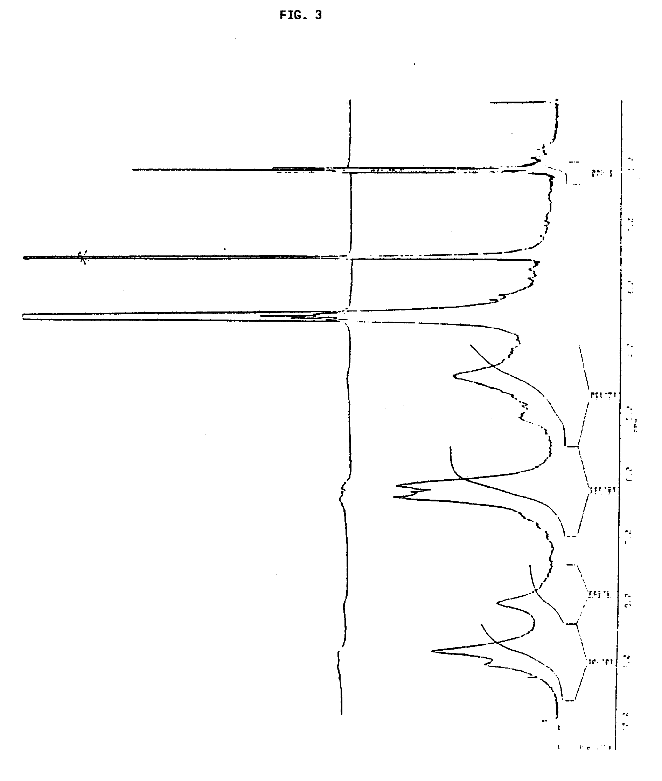

b.3. spectre de résonance magnétique protonique: voir figure 3

Instrument: Bruker HX-90 E, avec diméthylsulfoxyde comme modèle de référence.

Solvant: $D_2O$ RMN $^1H$ $\delta$ (ppm): 1,13 (multiplet, ci-après par abréviation "m"); 3,36 (m); 4,33 (singlet, ci-après par abréviation "s"); 5,1 (s); 6,24 (doublet, ci-après par abréviation "d"); 8,01 (s); 9,01 (s); 9,98 (s).

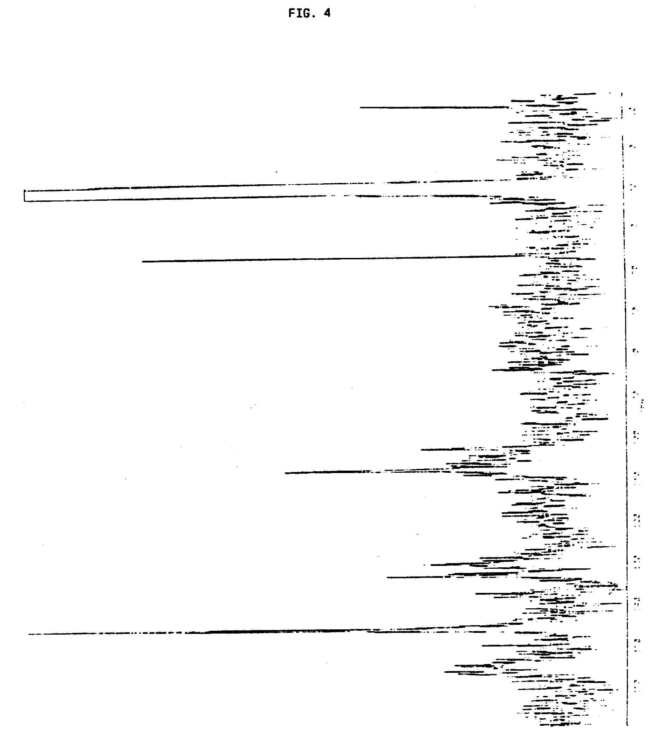

b.4. spectre de résonance magnétique $^{13}C$ : voir Figure 4

Instrument : Bruker HX-90 E avec TMS comme modèle interne

Solvant $D_2O$

RMN $^{13}C$ $\delta$ (ppm)

17,5, 18,25, 18,75, 19,5, 20,02, 21, 23, 25, 25,5, 26,25, 27,5, 27,9, 32, 32,8, 33,56, 36,4, 36,52, 37,5, 42, 42,2, 43, 45,2, 47,2, 49,2, 50,2, 52, 54, 55, 59,5, 60, 60,2, 62,7, 64,7, 65, 67,2, 68, 68,1, 69, 69,9, 70,5, 71,7, 74,2, 75, 76, 76,5, 77, 78,2, 81,2, 82,5, 83, 89, 96, 98, 98,7, 100, 102, 103,2, 103,5, 105, 105,2, 105,5, 106,5, 107,5, 109,7, 112, 112,2, 112,5, 112,7, 115, 115,7, 117,5, 118,5, 119,7, 123,5, 127, 128, 128,8, 129, 130, 131,5, 132, 136,9, 137,5, 143, 144,2, 144,7, 144,5, 146,2, 149,5, 150,5, 151, 152,7, 153,5, 153,6, 153,7, 155,5, 156, 157, 158, 160, 164, 163, 166, 167. On lit seulement jusqu'à 170.

c) caractéristiques chimiques :

c.1. analyse élémentaire :

C ; 54-57%

H ; 4,5-5,4%

0 : 36,5-37,5%

c.2. réactions chimiques :

| A) Réactif | Résultat |
|---|---|
| chlorure ferrique à 1% | couleur café verdâtre |
| acétate de plomb à 1% | précipité blanc |
| eau de brome | produit une décoloration |
| acide picrique | couleur jaune paille |
| ninhydride | négatif |
| fluoroglucinol | négatif |

B) Hydrolyse acide avec l'acide sulfurique

On met une solution de 250 mg du principe actif dans 10 ml d'acide sulfurique 2N, dans une petite ampoule de verre, puis on scelle et on chauffe au bain marie pendant 1 heure ½. La préparation des produits d'hydrolyse se fait par chromatographie en colonne sèche en employant comme support le gel de silice de 0,210-0,062 mm d'ouverture de maille (Merck). On utilise comme éluant : CCl$_4$-acétate d'éthyle-éthanol (4:2:1 V/V) et on obtient de bas en haut les fractions suivantes :

1.- couleur rose

2.- couleur jaune

3.- couleur rouge foncé

4.- couleur rouge clair

5.- couleur rouge foncé

qui présentent les caractéristiques physiques suivantes :

- point de fusion supérieur à 300°C

- solubles dans l'éthanol, le dimétylsufoxyde, l'acétone, le méthanol

- insolubles dans CCl$_4$, le benzène, l'acétate d'éthyle.

On réalise ensuite l'étude spectroscopique des fractions obtenues. On effectue le spectre IR sur un comprimé de KBr, et pour la détermination de RMN on emploie le dimétylsulfoxyde. Les résultats obtenus sont:

Fraction 1

EP 0 349 469 B1

IR, bandes d'absorption : 3380, 2900, 1630, 1520, 1380, 1220, 1060, 1010, 920, 780, 620, 580, 480, 430 cm$^{-1}$.

RMN $^1$H : 1,042 (t), 3,44 (quadruplet), 3,76 (d), 5,02 (s), ppm.

Fraction 2

IR, bandes d'absorption : 3290, 2910, 1630, 1530, 1440, 1210, 1110, 1030, 760 cm$^{-1}$.

RMN $^1$H : 1,22 (s), 3,30 (s), 6,5 (m), 8,78 (m), ppm.

Fraction 3

IR, bandes d'absorption : 3290, 2910, 1620, 1520, 1430, 1360, 1000, 760 cm$^{-1}$.

RMN $^1$H : 0,85 (s), 1,10 (s), 1,22 (s), 3,15 (s), 3,40 (s), 3,70 (s), 4,13 (s), 4,56 (s), 7,68 (s), 7,83 (s), ppm.

Fraction 4

IR, bandes d'absorption : 3290, 2980, 1710, 1540, 1470, 1450, 1430, 1380, 1230, 1110, 1070, 1020, 940, 800, 620, 580, 490, 430 cm$^{-1}$.

Fraction 5

Ir, bandes d'absorption : 3290, 2920, 1610, 1520, 1440, 1190, 1110, 1020, 830, 660, 650 cm$^{-1}$.

Ensuite, on dérive ces fractions pour obtenir les acétates correspondants. Pour cela, on prend 100 mg ce chaque fraction, sauf de la fraction 3 puisque la quantité obtenue de l'hydrolyse acide était très faible. On dissout dans le dimétylsulfoxyde et on ajoute 3 ml d'anhydride acétique et 1,5 ml de pyridine. On laisse au répos pendant 5 jours, après quoi on ajoute 5 ml d'eau distillée. On porte ensuite à pression réduite et on dissout le résidu obtenu dans le dimétylsulfoxyde puis on chromatographie en colonne sèche en employant comme support le gel de silice de 0,210-0,062 mm d'ouverture de maille (Merck), et comme éluants, on emploie les systèmes suivants :

a) pour l'acétate de la première fraction :

acétone-chloroforme 2:1 V/V

b) pour les acétates des fractions 2, 4 et 5 :

chloroforme-méthanol 3:2 V/V

Etude spectroscopique. Pour les composés acétylés on prend les spectres IR sur des comprimés de KBr. Pour la détermination de RMN, on emploie le diméthylsulfoxyde deutérié.

Fraction 1

RMN$^1$H (dimétylsulfoxyde comme référence): 1,3 (t), 1,94 (s), 2,03 (s), 3,4 (d), 3,43 (s), 3,69 (m), 8,1 (m), 8,59 (t), 8,93 (s) ppm.

RMN $^{13}$C : 15,27, 18,68, 20,7, 56,2, 61,7, 127,5, 142,3, 146,6 ppm.

RMN, (avec chloroforme comme référence) : 0,9 (s), 1,3 (s), 1,6 (s), 2,0 (s), 2,2 (s), 2,35 (t), 2,55 (s), 7,5 (m), 7,63 (m) ppm.

Fraction 2

RMN$^1$H (dimétylsulfoxyde comme référence): 1,15 (d), 1,20(s), 1,9 (s) 2,06 (s), ppm.

Fraction 5

RMN$^1$H (dimétylsulfoxyde comme référence): 1,12(m), 2,02(m), ppm.

c) hydrolyse acide avec acide periodique 2N.

On soumet à une température de reflux pendant 1 heure ½ une solution de un gramme de principe actif dans 50 ml d'acide periodique 2N. La séparation des produits d'hydrolyse se fait par chromatographie en colonne sèche en employant comme support le gel de silice de 0,210-0,062 mm d'ouverture de maille (Merck). On emploie comme éluants les systèmes suivants :

a). chloroforme-méthanol 3:2 V/V

b). tétrachlorure de carbone-acétate d'éthyle-éthanol 4:2:1 V/V.

On obtient de bas en haut les fractions suivantes :

COULEUR

1.- violet

2.- jaune prononcé

3.- jaune paille

4.- café clair

5.- café foncé

Etude spectroscopique. Pour les composés obtenus, on prend les spectres IR sur comprimés de KBr. Pour la détermination de RMN on emploie le dimétylsufoxyde dutérié.

Fraction 1

RMN$^1$H (diméthylsulfoxyde comme référence): 0,98 (m), 1,22 (s), 2,09 (s), ppm.

Fraction 2

(chloroforme comme référence)

6

IR, bande d'absorption : 2922, 2820, 1727, 1702, 1625, 1933, 1384, 1130, 1064 cm$^{-1}$.

RMN $^1$H : 0,83 (s), 1,035 (t), 1,22 (s), 3,35 (s), 3,48 (s), 3,63 (s), ppm.

Fraction 3

IR, bande d'absorption : 2820, 1740, 1610, 1440, 1130, 1070, 1000, 920 cm$^{-1}$.

RMN$^1$H (dimétylsulfoxyde comme référence): 0,85(s), 1,05 (t), 1,23 (s), 3,16 (s), 3,43 (d), 3,63 (s), 4,15 (s), 4,40 (s), 7,39 (m) ppm.

Fraction 4

(dimétylsulfoxyde comme référence)

RMN $^1$H : 0,8 (m), 1,05 (s), 1,24 (s), 1,51 (s), 3,41 (s), 3,65 (s), 4,30 (s), 4,41 (s), 7,46 (m), 8,40 (m), ppm.

Fraction 5

(diméthylsulfoxyde comme référence)

Ir, bande d'absorption : 2953, 1742, 1620, 1439, 1234, 1130, 1084, 900, 830 cm$^{-1}$.

RMN $^1$H : 1,03 (t), 1,47 (d), 3,16 (s), 3,40 (quadruplet), 3,63 (s), 4,22 (s), 4,26 (s), 4,39 (s), 7,45 (s) ppm.

L'invention se réfère à un procédé d'extraction et d'isolement du principe actif caractérisé ci-dessus, contenu dans l'écorce de Mimosa tenuiflora, et doté d'une activité de régénération cellulaire.

Ledit procédé consiste essentiellement en une extraction successive au moyen de solvants.

Dans une première étape, on pulvérise ou on broie l'écorce de Mimosa tenuiflora de préférence au moyen d'un moulin à ailettes en obtenant une mouture que l'on met dans un réacteur où l'on procède à une extraction préalable avec du chloroforme en une proportion comprise entre 2:1 et 8:5 P/V (parties en poids de mouture : parties en volume de chloroforme), en chauffant à une température comprise approximativement entre 50°C et 70°C, dans le but d'éliminer les alcaloïdes, les graisses et autres composés chimiques présents dans l'écorce. Ensuite, on sépare la fraction chloroformique, de préférence au moyen d'une filtration en utilisant un type de filtre presse.

Dans une seconde étape, sur la pâte obtenue après la séparation de la phase chloroformique, on effectue une seconde extraction avec de l'éthanol en une proportion comprise entre 1:3 et 1:12 P/V respectivement à une température comprise entre environ 60°C et 80°C tout en agitant de façon continue. De cette manière, on obtient le principe actif en solution alcoolique. Ensuite on filtre et on élimine l'éthanol par distillation et on sèche le résidu obtenu dans une armoire étuve. On peut répéter cette étape plusieurs fois en recueillant et en réunissant les extraits éthanoliques.

Ensuite, dans une troisième étape, on soumet le résidu sec à une nouvelle extraction avec de l'eau en une proportion de 1:2 P/V respectivement, à une température comprise entre environ 50°C et 100°C en agitant de façon continue. De cette manière on obtient une solution aqueuse du principe actif que l'on sépare par filtration. On peut répéter plusieurs fois cette étape d'extraction avec de l'eau et on recueille les solutions aqueuses obtenues. On laisse au repos les solutions aqueuses réunies à la température ambiante entre 12 et 92 heures. Ensuite, on effectue une concentration préalable du principe actif par évaporation partielle de l'eau. Pour cela, on répète la filtration des solutions aqueuses du principe actif et on fait évaporer partiellement l'eau jusqu'à obtenir une concentration de 20 % en solides.

Enfin, on les met dans une armoire étuve et on sèche en obtenant le principe actif sous la forme d'une poudre de couleur café rougeâtre à saveur amère et inodore. L'analyse élémentaire du principe actif extrait indique que la proportion de C, H et O est la suivante :

C : 54-57%

H : 4,5-5,4%

O : 36,5-37,5%

L'identification de ce composé se fait au moyen d'une spectroscopie ultraviolette/visible, infrarouge et de Résonance Magnétique Nucléaire, en obtenant des spectres similaires à ceux qui sont reportés dans les Figures 1 à 4 de la présente demande, et dont les caractéristiques ont été mentionnées plus haut.

Selon le procédé, on peut préparer le principe actif avec un rendement qui oscille entre 9 et 11% approximativement, la pureté du produit ainsi obtenu étant adéquate pour les objectifs pharmaceutiques.

Alternativement, on peut également obtenir le principe actif au moyen d'une extraction avec un mélange d'éthanol-eau dans une proportion de 1:1 (V/V), suivie d'une élimination de l'éthanol par distillation et ensuite on peut effectuer une nouvelle extraction avec du chloroforme sur la solution aqueuse dans le but d'assurer la purification. Ensuite, on porte à siccité la solution aqueuse résultante après élimination du chloroforme pour obtenir le principe actif sous la forme d'une poudre ayant les caractéristiques mentionnées plus haut.

Les solvants cités dans les extractions mentionnées précédemment ne sont pas critiques, bien qu'ils soient préférés. Cependant, on peut également utiliser d'autres solvants comme le méthanol, l'acétone, l'acétate d'éthyle, l'éther éthylique, le diméthylsulfoxyde, l'hexane, le benzène, le tétrachlorure de carbone, le tétrahydrofuranne ou l'éther de pétrole. Comme techniques d'extraction, on peut utiliser aussi bien le procédé de lixiviation-percolation, que la macération ou l'extraction continue ou une autre technique analogue bien connue des spécialistes de la question.

L'invention se réfère également aux compositions pharmaceutiques qui contiennent le principe actif extrait de l'écorce de Mimosa tenuiflora avec activité de régénération épidermique obtenu par le procédé décrit. Ces compositions sont utiles pour le traitement des brûlures et pour le traitement de lésions d'une autre nature comme les ulcères, varices, blessures chirurgicales, lésions légères, crevasses, éraflures, écorchures, plaies et eczéma.

Ces compositions pharmaceutiques peuvent se présenter sous forme solide ou liquide. Sous forme solide, elles peuvent se présenter en poudres, poudres saupoudrables, onguents, etc. Sous forme liquide, elles peuvent se présenter en émulsions, suspensions, gelées, shampooings, etc. Les suspensions ou émulsions peuvent de même être converties en aérosols.

Les compositions pharmaceutiques contiennent une quantité, en général, de principe actif comprise entre environ 1 et 70% en poids, jointe à une quantité adéquate de véhicules et excipients appropriés.

Les compositions pharmaceutiques préférées sont la solution aqueuse et la poudre saupoudrable.

La posologie du principe actif dépend de la surface de la lésion et de sa profondeur. A l'application typiquement sur la zone blessée, il forme une pellicule de 0,02 à 0,1 mm d'épaisseur.

L'étude comparative du pouvoir de régénération épidermique de l'écorce en poudre de Tepescohuite et de son principe actif, a été faite sur 48 rats (Rattus Norve gicus) atteints de brûlures du second degré sur 20% de la superficie corporelle. On effectue également une évaluation sur 80 malades humains atteints de brûlures du second degré, superficielles ou profondes, avec une superficie corporelle de 10 à 40%.

Avec la poudre de principe actif on prépare une série de solutions qui présentent un intervalle de concentration allant de 5 à 60%, car les avantages de la solution de principe actif sur la poudre d'écorce de Tepescohuite sont les suivants :

La solution lors de son application a cet avantage qu'elle se moule et adhère à la totalité de la blessure sans laisser de vide, produisant une couche protectrice qui sèche rapidement, permettant les échanges gazeux.

Ainsi, elle exerce une action desséchante, recouvre les terminaisons nerveuses, ainsi que les couches cellulaires de la peau qui restent également dépourvues de projections lors de la lésion de l'épiderme, réduit la durée de la douleur, diminue l'inflammation, empêche l'entrée des microorganismes, évite l'activité pyrogène et algogène que produit l'écorce en poudre en évitant ainsi la présence d'un prurit, et en éliminant la déperdition de liquide diminue le déséquilibre hydrostatique.

Elle conserve le pH acide de la peau traumatisée, présente une plus grande mobilité des extrémités, active comme un puissant inducteur du tissu de granulation.

Elle présente une puissante action angionique qui favorise la néoformation artérielle et la vasodilatation et active comme chimiotactique la population des macrophages.

C'est un puissant inducteur de la collagénogénèse de la part les fibroblastes. Elle diminue par conséquent l'alignement des fibres de collagène dans la direction des lignes de traction et ainsi la formation de cicatrices hypertrophiques et chéloïdes.

Elle induit la régénération de la peau plus rapidement que dans le cas de l'écorce en poudre.

Pour la quantification de la toxicité aiguë et subaiguë du principe actif de l'invention, on utilise des rats blancs (Rattus Norve gicus) et de souche Wistar Lewis des deux sexes, avec un poids moyen de 350 g pour les mâles et de 275 g pour les femelles. On teste le composé par voie sous-cutanée.

Dans l'étude de la toxicité aiguë, on n'obtient le paramètre toxicologique $DL_{50}$ à aucune dose essayée (0, 100, 177, 316, 562 ; 1000, 1778, 3162 mg/kg), puisque la valeur maximale de l'indice de mortalité atteint est de 10%, pour une DL (dose létiphère) de 3162mg/kg, pour un temps d'observation de 14 jours, ce qui fait que la valeur moyenne de $DL_5$ est de 650 mg/kg.

Dans la toxicité subaiguë, on obtient une valeur de $DL_{50}$ de 9,759 g/kg avec un temps d'observation de 32 jours, la valeur de la concentration de la $DL_{50}$ dans ce cas descendant à 520 mg/kg.

L'étude histopathologique est faite sur la peau, le rein, le foie, l'intestin, le muscle, en employant les techniques classiques de fixation et de teinture des tissus par Hematoxilyne-Eosine. On rencontre sur la peau de grands dépôts de matière ocre, amorphe, présentant une intense réaction granulomateuse et de type corps étranger. On note également des macrophages et des fibroblastes en grand nombre et une production élevée de collagène.

Cette étude porte à penser que la matière présente un bas taux d'absorption et que le principe actif peut être considéré comme atoxique, puisqu'il serait nécessaire d'avoir des concentrations de 585 mg/kg pour avoir une probabilité té de mortalité de 50% pour une personne adulte de 60 kg en lui inoculant le principe actif par voie sous-cutanée.

Chez le malade, on emploie la voie locale car elle diminue considérablement la toxicité.

L'étude clinique du principe actif a été faite chez 50 malades brûlés, qui se subdivisent en 4 groupes selon l'importance de la superficie affectée :
- groupe I moins de 10%
- groupe II de 11 à 20%
- groupe III de 21 à 30%
- groupe IV plus de 31%

dans le groupe I la tension artérielle a été évaluée en 3 occasions et on a observé dans la TA systolique (mm Hg) les valeurs suivantes $\underline{x}+S$ ; 112,85+17,04 ; 117,5+17,08 et 117,5-20,6 respectivement. Quant à la TA diastolique, les valeurs sont 78±21,68 ; 72,5±12,58 et 76,66±15,27. Les valeurs de la TA tant systoliques que diastoliques ne montrent pas de différences statistiques significatives.

Le pouls artériel (fréquence par minute) est de 87,43±13,34 ; 88±8,07 ; 90,25±7,93 et 80,5±8,22, valeurs similaires sans différences statistiques significative.

Quant à la fréquence respiratoire (par minute), on observe 25,7±7,16 ; 23±3,74 ; 22,75±2,5 et 22,5±2,08 également avec un P non significatif.

La température moyenne matinale du corps est de 36,57±0,62 ; 36,75±0,98 ; 36,55±1,02 et 36,77±0,82. Les données pour le soir sont les suivantes : 37,11±0,678 ; 36,85±0,2 ; 36,27±0,26 et 36,63±0,7, avec une différence significative entre le premier jour (température élevée) et le septième jour (température plus basse) où P<0,05.

La biométrie hématique montre : Hémoglobine (Hb) g/dl $\bar{x}$ ± S ; 13,58±1,39 et 13,45±0,86 Hématocrite (Ht)% $\bar{x}$ ±S ; 91,38±5,55 ; 41,0±4,74 Leucocytes $(1 \times 10^3 mm^3)$ 10936±3110 et 8683±962.

Dans toutes ces données, on n'observe pas de différence significative, et l'on n'a pas non plus de différence dans le flux urinaire (ml /24 heures)$\bar{x}$±S ; 705±327,14 ; 836,66±433,11 ; 785±513,71 et 782,5±382,7.

Lorsqu'on analyse les symptomes de sécrétion séreuse, de sécrétion hématique, de sécrétion purulente, de douleur, de prurit, d'ardeur, d'inflammation, d'infection, de granulation et de cicatrisation dans la première semaine de suivi on observe une amélioration et des différences significatives à partir du troisième jour et on note le maintien à la fin de la semaine des symptomes de sécrétion séreuse (P 0,001) ; sécrétion hématique (P 0,05), douleur (P 0,001), ardeur (P 0,001, inflammation (P 0,01), granulation (P 0,001), et cicatrisation (P 0,01) ce qui n'est pas le cas pour la sécrétion purulente et le prurit. Dans les groupes II, III et IV, on obtient des résultats analogues.

De tout cela on déduit que le principe actif extrait de l'écorce de Mimosa tenuiflora a des propriétés de régénération épidermique, qu'il est atoxique, qu'il ne produit pas d'effets secondaires, diminue la douleur, l'ardeur, les sécrétions et stimule la cicatrisation, ce qui fait qu'il a une large application à des buts thérapeutiques.

On trouvera ci-dessous quelques exemples d'extraction du principe actif ainsi que de préparation de compositions pharmaceutiques, dans des buts uniquement de précision et non de limitation de la portée de l'invention.

EXEMPLE 1

On sèche et on broie l'écorce de Tepescohuite dans un moulin à ailettes. On met 50 kg de cette écorce pulvérisée dans un réacteur de capacité suffisante, on ajoute du chloroforme en une proportion de 2:1 à 8:5 P/V respectivement, à une température de 50 à 70°C pendant une période de 4 à 8 heures en agitant de façon continue. On sépare le chloroforme par filtration (en employant un filtre presse).

On extrait l'écorce pulvérisée avec de l'éthanol dans une proportion de 1:3 à 1:12 P/V respectivement à une température de 60 à 80°C en maintenant l'agitation. On filtre et on répète l'extraction avec de l'éthanol, on réunit les extraits éthanoliques et on élimine l'éthanol par distillation, on sèche le résidu dans une armoire étuve.

On extrait la poudre obtenue avec de l'eau en une proportion de 1:2 à 1:10 P/V respectivement à une température de 50 à 100°C en continuant d'agiter.

On répète l'extraction avec de l'eau et on réunit les solutions aqueuses, on filtre et on les laisse au repos à la température ambiante entre 12 à 92 heures.

On filtre à nouveau la solution aqueuse, on concentre jusqu'à une teneur de 20% en solides, on sèche dans une armoire étuve jusqu'à obtenir une poudre de couleur rouge, de saveur amère et inodore. De cette manière, on obtient un composé qui présente une analyse élémentaire de :

C 54 à 57%
H 4,5 à 5,4%
O 36,5 à 37,5%

L'identification de ce composé se fait au moyen de UV/VIS, IR, RMN.

EXEMPLE 2

On met 50 kg de l'écorce pulvérisée dans un réacteur de volume suffisant. On y ajoute un mélange d'eau-éthanol 1:1 V/V, la relation de la solution alcoolique avec le poids d'écorce de Tepescohuite va de 4 à 1 à 1, respectivement. On chauffe ce mélange à une température de 60 à 95°C pendant une période de 3 à 12 heures en agitant continuellement, on sépare la solution éthanolique par filtration (filtre presse).

Avec l'écorce on répète le même procédé, on réunit les extraits eau-éthanol et on élimine l'éthanol par distillation, on laisse au repos pendant 12 à 96 heures. On filtre et on extrait la solution aqueuse avec du chloroforme en agitant énergiquement pour assurer la purification.

On concentre la solution aqueuse jusqu'à une teneur de 20% en solides. Ensuite, on porte à siccité dans une armoire étuve.

EXEMPLE 3

On répète le procédé de l'exemple 1, mais en remplaçant le chloroforme par l'alcool éthylique et l'éthanol par le méthanol.

EXEMPLE 4

On prépare une composition pharmaceutique du principe actif sous forme d'onguent qui a la composition suivante :

ONGUENT

| | |
|---|---|
| poudre de Tepescohuite | 10 g |
| beurre de cacao | 10 g |
| cire de carnauba | 5 g |
| huile végétale d'olive | 20 g |
| lanoline hydrogénée | 10 g |
| Tween-30 (R) | 1 g |
| Span-80 (R) | 1 g |
| p-hydroxybenzoate de méthyle | 1 g |
| eau distillée q.s.p. | 100 g |

On fait fondre lentement le beurre de cacao, l'huile d'olive, la cire de carnauba, la lanoline hydrogénée réunis au Tween-80 (R) et au Span-8- (R) à 80° C tout en agitent lentement de façon continue (phase lipophile).

On dissout le p-hydroxybenzoate de méthyle et l'extrait de Tepescohuite dans de l'eau distillée en chauffant à environ 60°C.

On ajoute la solution aqueuse chauffée à la phase lipophile en maintenant la température entre 60 et 70°C en agitant de façon continue pendant 15 minutes puis une fois le tout bien homogénéisé on l'introduit dans les flacons respectifs.

EXEMPLE 5

On prépare un hydrogel du principe actif qui a la composition suivante :

<u>HYDROGEL</u>

```
poudre de Tepescohuite                    6 g
Carbopol®934(polymère de car-
boxyvinyle)                               0,9 g
triéthanolamine                           1 g
P-hydroxybenzoate de méthyle              0,1 g
isopropanol                               32 g
eau distillée q.s.p.                      100 g
```

On dissout le p-hydroxybenzoate de méthyle dans une partie d'eau distillée. On fait gonfler le Carbopol®934 dans la solution obtenue et on neutralise ensuite avec la triéthanolamine. Après obtention d'une suspension de la substance active avec de l'isopropanol, on ajuste le volume avec de l'eau distillée jusqu'à 100 g.

## EXEMPLE 6

On prépare un gel du principe actif ayant la composition suivante :

<u>GEL</u>

```
poudre de Tepescohuite
(solution aqueuse à 20%)                  8 g
Carbowax 6000 (éther poly
(éthylèneglycol)monoalcoylique            15 g
Carbowax 300 (éther poly(éthy-
lèneglycol)monoalcoylique)                30 g


Carbowax 400 (éther poly-éthy-
lèneglycol)monoalcoylique)                27 g
glycérol                                  18 g
Brij35 (éther poly(éthylène-
glycol)laurylé)                           2 g
```

On fait fondre ensemble les composants, à l'exception de la substance active, on ajoute à celle-ci la masse fondue en petites fractions tout en agitant et on homogénéise la composition.

## EXEMPLE 7

On prépare une poudre saupoudrable du principe actif ayant la composition suivante :

POUDRE SAUPOUDRABLE

| | |
|---|---|
| poudre de Tepescohuite | 14 g |
| stéarate de zinc | 5 g |
| oxyde de zinc | 4,5 g |
| Aerosil® R-200 | 3,8 g |
| talc q.s.p. | 100 g |

On homogénéise minutieusement les composants pour obtenir un mélange en poudre.

EXEMPLE 8

On prépare un baume pour les lèvres contenant le principe actif avec la composition suivante :

BAUME POUR LEVRES A BASE DE TEPESCOHUITE

Composition centésimale

| | |
|---|---|
| extrait de Tepescohuite | 10 g |
| alcool cétylique | 5 g |
| cire blanche d'abeille en grumeaux | 12 g |
| lanoline | 2 g |
| vaseline filante | 10 g |
| stéarate d'éthyle | 8 g |
| cire de carnauba | 10 g |
| acide benzoïque | 0,5 g |
| Spand | 0,5 g |
| huile de vaseline industrielle | 32 g |
| eau q.s.p. | 100 g |

On fait fondre à une température d'environ 80°C la lanoline, les cires, le stéarate d'éthyle et le Spand joint à l'huile de vaseline, en remuant la pâte lentement pendant une période de 30 minutes (phase lipophile).

On dissout l'acide benzoïque et l'extrait de Tepescohuite dans l'eau distillée en chauffant à environ 100°C.

Ensuite, on ajoute la solution aqueuse à la phase lipophile en agitant continuellement pendant encore 30 minutes et on verse dans des moules préalablement préparés.

EXEMPLE 9

On prépare une solution aqueuse du principe actif ayant la composition :

SOLUTION

| | |
|---|---|
| poudre de principe actif de Tepescohuite | 20 g |
| eau distillée | 80 g |

On dissout la poudre de principe actif dans de l'eau en chauffant à 70°C.

## Revendications

1. Procédé pour l'obtention du principe actif de l'écorce de <u>Mimosa tenuiflora</u>, aux caractéristiques suivantes:
   a) il s'agit d'une poudre de couleur café rouge, de saveur amère et inodore.
   b) il y a un point de fusion de 222º C.
   c) il est soluble dans l'éthanol, le dimétylsulfoxyde, le méthanol et l'acétone.
   d) il est insoluble dans l'eau, le chloroforme, l'acétate d'éthyle, l'éther éthylique, le propanol, l'hexane, $CCl_4$, le tétrahydrofurane, le petrolatum, le dioxanne, l'alcool amylique, l'alcool isopropylique, l'alcool benzylique et la diéthylamine.
   e) en chromatographie en couche mince il présente les valeurs suivantes de R avec les systèmes d'élution suivants:

| Système d'élution | Relation frontale (Rf) |
| --- | --- |
| chloroforme | 0 |
| acétate d'éthyle | 0 |
| éther de pétrole | 0 |
| benzène | 0 |
| méthanol-eau-**dimétylsulfoxyde**(1:1:1)V/V | 0,963 |
| éthanol-eau-acide acétique (2:1:1:) V/V | 0,817 |
| méthanol-**dimétylsulfoxyde** | 0,910 |
| **dimétylsulfoxyde** | 1,000 |
| dioxanne-méthanol-acétate d'éthyle (1:1:1) V/V | 0 |

   f) il ne présente pas d'éléments lourds comme Pb, Zn, Cr, Fe, Mn, Cu, Hg ou As.
   g) il présente un spectre d'absorption ultraviolette en solution éthanolique, avec des maximum d'absorption et d'absorbance de 222 mm (a=2,840) et 281 mm (a=0,600).
   h) il présente un spectre d'absorption IR (sur comprimé de bromure de potassium) avec des bandes spectroscopiques d'absorption à: 3290, 2910, 1630, 1530, 1450, 1190, 1150, 1110, 1020, 840, 760, 620, 580 cm$^{-1}$
   i) il présente un spectre d'absorption de RMN protonique dans $D_2O$ avec des signaux à: 1,13 (multiplet), 3,36 (multiplet), 4,33 (singlet), 5,1 (singlet), 6,24 (doublet), 8,01 (singlet), 8,81 (singlet), 9,01 (singlet), 9,98 (singlet) ppm;
   j) il présente un spectre de RMN $^{13}C$ dans $D_2O$ avec des signaux à :
   17,5, 18,25, 18,75, 19,5, 20,02, 21, 23, 25, 25,5, 26,25, 27,5, 27,9, 32, 32,8, 33,56, 36,4, 36,52, 37,5, 42, 42,2, 43, 45,2, 47,2, 49,2, 50,2, 52, 54, 55, 59,5, 60, 60,2, 62,7, 74,7, 65, 67,2, 68, 68,1, 69, 69,9, 70,5, 71,7, 74,2, 75,76, 76,5, 77, 78,2, 81,2, 82,5, 83, 89, 96, 98, 98,7, 100, 102, 103,2, 103,5, 105, 105,2, 105,5, 106,5, 107,5, 109,7, 112, 112,2, 112,5, 112,7, 115, 115,7, 117,5, 118,5, 119,7, 123,5, 127, 128, 128,8, 129, 130, 131,5, 132, 136,9, 137,5, 143, 144,2, 144,7, 144,5, 146,2, 149,5, 150,5, 151, 152,7, 153,5, 153,6, 153,7, 155,5, 156, 157, 158, 160, 164, 163, 166, 167. (on ne lit que jusqu'à 170).
   k) l'analyse élémentaire présente les proportions suivantes :
   C : 54-57%
   H : 4,5-5,4%
   0 : 36,5-37,5%
   caractérisé en ce que:
   a) on broie ou on moud l'écorce de <u>Mimosa tenuiflora</u> jusqu'à obtenir une mouture,
   b) on traite ladite mouture avec un solvant en une proportion de 2:1 à 8:5 P/V respectivement, en chauffant et on sépare le solvant par filtration, en obtenant un résidu qu'ensuite
   c) on traite avec un autre solvant dans une proportion de 1:3 à 1:12 P/V respectivement, en chauffant et en agitant de façon continue, pour ensuite éliminer lesdits solvants par distillation, en obtenant un résidu que l'on sèche; cette étape pouvant être répétée plusieurs fois; et

d) on la soumet à une troisième extraction avec de l'eau en une proportion de 1:2 à 1:10 P/V respectivement, en chauffant et en agitant, cette étape pouvant être réalisée plusieurs fois, en recueillant et en séparant les solutions aqueuses par filtration et en les laissant au répos pendant 12 à 92 heures, pour ensuite,.

e) on concentre la solution aqueuse jusqu'à 20% de solides par élimination de l'eau, puis mettre dans une étuve jusqu'à siccité en obtenant le composé désiré sous forme de poudre.

2. Procédé selon la revendication 1, caractérisé en ce que:
- on réalise l'étape b) avec du chloroforme comme solvant dans un intervalle de températures compris entre 50 et 70º C.
- on réalise l'étape c) avec de l'éthanol comme solvant et dans un intervalle de températures compris entre 60 et 80º C.
- on réalise l'étape d) avec de l'eau comme solvant et dans un intervalle de températures compris entre 50 et 100º C.

3. Procédé selon les revendications 1 et 2, caractérisé en ce que les solvants à utiliser dans l'extraction sont choisis entre le méthanol, l'acétone, l'acétate d'éthylique, le dimétylsulfoxyde, l'hexane, le benzène, $CCl_4$, le tétrahydrofurane ou l'éther de pétrole.

4. Procédé selon une des revendications 1 à 3, caractérisé en ce qu'on ajoute des excipients ou véhicules pour obtenir une composition pharmaceutique.

5. Procédé selon la revendication 4, caractérisé en ce que la quantité de principe actif varie entre 1 et 70% en poids.

6. Procédé selon la revendication 4, caractérisé en ce que la composition pharmaceutique se présente sous forme solide ou liquide.

7. Procédé selon la revendication 4, caractérisé en ce que la composition pharmaceutique se presente sous forme de solution aqueuse ou de poudre à saupoudrer.


**Patentansprüche**

1. Verfahren für den Erhalt des Wirkstoffes der Rinde der Mimosa Tenuiflora (Mimose) mit folgenden Eigenschaften:
a) es handelt sich um eine rotbraune, geruchlose Substanz in Puderform mit einem bitteren Geschmack
b) der Schmelzpunkt liegt bei 222ºC
c) die Substanz ist lösbar in Äthanol, Dimethylsulfoxyd, Methanol und Aceton.
d) die Substanz ist nicht lösbar in Wasser, Chloroform, Äthylacetat, Äthyläther, Propanol, Hexan, CCl4, Tetrahydrofuran, Petrolat, Dioxan, Amylalkohol, Isopropylalkohol, Benzylalkohol und Diethylamine.
e) sie weist bei der dünnlagigen Chromatographie folgende Werte für R mit nachstehenden Elutionssystemen auf:

| Elutionssysteme | Frontalverhältnis (Rf) |
|---|---|
| Chloroform | 0 |
| Äthylacetat | 0 |
| Petroläther | 0 |
| Benzol | 0 |
| Methanolwasserdimethylsulfoxyd (1:1:1) V/V | 0,963 |
| Äthanol-Wasser-Essigsäure (2:1:1:) V/V | 0,817 |
| Methanol-Dimethylsulfoxyd | 0,910 |
| Dimethylsulfoxyd | 1,000 |
| Äthyldioxan-Methanol-Acetat (1:1:1) V/V | 0 |

f) die Substanz weist keine Schwermetalle auf wie z.B. Pb, Zn, Cr, Fe, Mn, Cu, Hg noch As.

g) die Substanz weist ein ultraviolettes Absorptions-spektrum in einer Äthanollösung auf mit einem Höchstabsorptions- und Absorbierungswert von 222 mm (a=2,840) und 281 mm (a=0,600).

h) das IR-Absorptionsspektrum weist (auf gepresstem Kaliumbromid) folgende spektroskopische Absorptionsbänder auf : 3290, 2910, 1630, 1530, 1450, 1190, 1150, 1110, 1020, 840, 760, 620, 580 $cm^{-1}$.

i) das Protonen-NMR-Absorptionsspektrum in $D_2O$ weist folgende Signale auf: 1,13 (mehrfach), 3,36 (mehrfach), 4,33 (einfach), 5,1 (einfach), 6,24 (doppelt), 8,01 (einfach), 8,81 (einfach), 9,01 (einfach), 9,98 (einfach) ppm;

j) das $^{13}C$ NMR-Spektrum in $D_2O$ weist folgende Signale auf:
17,5, 18,25, 18,75, 19,5, 20,02, 21, 23, 25, 25,5, 26,25, 17,5, 27,9, 32, 32,8, 33,56, 36,4, 36,52, 37,5, 42, 42,2, 43, 45,2, 47,2, 49,2, 50,2, 52, 54, 55, 59,5, 60, 60,2, 62,7, 74,7, 65, 67,2, 68, 68,1, 69, 69,9, 70,5, 71,7, 74,2, 75, 76, 76,5, 77, 78,2, 81,2, 82,5, 83, 89, 96, 98, 98,7, 100, 102, 103,2, 103,5, 105, 105,2, 105,5, 106,5, 107,5, 109,7, 112, 112,2, 112,5, 112,7, 115, 115,7, 117,5, 118,5, 119,7, 123,5, 127, 128, 128,8, 129, 130, 131,5, 132, 136,9, 137,5, 143, 144,2, 144,7, 144,5, 146,2, 149,5, 150,5, 151, 152,7, 153,5, 153,6, 153,7, 155,5, 156, 157, 158, 160, 164, 163, 166, 167 (ablesbar nur bis 170).

k) die Elementaranalyse weist folgendes Verhältnis auf:
C : 54 - 57 %
H : 4,5 - 5,4 %
O : 36,5 - 37,5 %
dadurch gekennzeichnet, dass:

a) die Rinde der Mimosa Tenuiflora wird zerkleinert und gemahlen, bis eine feingemahlene Mischung hergestellt ist;

b) diese Feinmischung wird unter Erhitzung mit einem Lösungsmittel in einem Verhältnis von je 2:1 bis 8:5 M/V aufbereitet, wonach das Lösungsmittel durch Filterung abgetrennt wird; der Rückstand wird danach wie folgt verarbeitet:

c) Aufbereitung mit einem anderen Lösungsmittel in einem Verhältnis von je 1:3 bis 1:12 M/V, unter Erhitzung und ständigem Rühren, wonach die erwähnten Lösungsmittel mittels Destillation abgetrennt werden; der Rückstand wird getrocknet; dieser Schritt kann mehrmals wiederholt werden; und

d) die Mischung wird einer dritten Extraktion mit Wasser unterworfen in einem Verhältnis von je 1:2 bis 1:10 P/V, unter Erhitzung und Rühren; dieser Schritt kann mehrmals wiederholt werden, indem die wässrigen Lösungen aufgefangen und mittels Filterung getrennt werden und man sie 12 bis 92 Stunden stillstehen lässt; anschließend

e) wird die wässrige Lösung auf 20% Festgehalt konzentriert, indem das Wasser entzogen wird, wonach sie in eine Trockenkammer gebracht wird bis zur vollständigen Entwässerung. Man erhält die gewünschte Verbindung in Staubform.

2. Verfahren gemäss Anspruch 1, dadurch gekennzeichnet, daß:

- bei Schritt b) als Lösungsmittel Chloroform benutzt wird und der Temperaturbereich zwischen 50 und 70ºC liegt;
- bei Schritt c) als Lösemittel Äthanol benutzt wird und der Temperaturbereich zwischen 60 und 80ºC liegt;
- bei Schritt d) als Lösungsmittel Wasser benutzt wird und der Temperaturbereich zwischen 50 und 100ºC liegt.

3. Verfahren nach den Ansprüchen 1 und 2, dadurch gekennzeichnet, daß die für die Extraktion zu verwendenden Lösungsmittel gewählt werden unter Methanol, Aceton, Äthylacetat, Dimethylsulfoxyd, Hexan, Benzol, CCl4, Tetrahydrofuran oder Petroläther.

4. Verfahren nach einem der Ansprüche 1 bis 3, dadurch gekennzeichnet, daß man Bindemittel oder Träger hinzufügt, um eine pharmazeutische Verbindung zu erhalten.

5. Verfahren nach Anspruch 4, dadurch gekennzeichnet, daß die Menge des Wirkstoffes zwischen 1 und 70 Gewichtsprozent schwankt.

6. Verfahren nach Anspruch 4, dadurch gekennzeichnet, daß die pharmazeutische Verbindung in fester oder flüssiger Form hergestellt werden kann.

7. Verfahren nach Anspruch 4, dadurch gekennzeichnet, daß die pharmazeutische Verbindung als wässrige Lösung oder als stäubbarer Puder vorliegt.


**Claims**

1. Process for obtaining the active principle of <u>Mimosa tenuiflora</u> bark, having the following characteristics:
   a) it is a bitter-tasting and odourless, coffee-red powder;
   b) it has a melting point of 222°C;
   c) it is soluble in ethanol, dimethyl sulphoxide, methanol and acetone;
   d) it is insoluble in water, chloroform, ethyl acetate, ethyl ether, propanol, hexane, $CCl_4$, tetrahydrofur-an, petrolatum, dioxane, amyl alcohol, isopropyl alcohol, benzyl alcohol and diethylamine;
   e) in thin-layer chromatography, it possesses the following R values with the following elution systems:

| Elution system | Substance/front ratio (Rf) |
|---|---|
| chloroform | 0 |
| ethyl acetate | 0 |
| petroleum ether | 0 |
| benzene | 0 |
| methanol/water/dimethyl sulphoxide (1:1:1 V/V) | 0.963 |
| ethanol/water/acetic acid (2:1:1 V/V) | 0.817 |
| methanol/dimethyl sulphoxide | 0.910 |
| dimethyl sulphoxide | 1.000 |
| dioxane/methanol/ethyl acetate (1:1:1 V/V) | 0 |

f) it does not contain heavy elements such as Pb, Zn, Cr, Fe, Mn, Cu, Hg or As;
g) it possesses an ultraviolet absorption spectrum in ethanolic solution with absorption and absorbance maxima at 222 nm (a=2.840) and 281 nm (a=0.600);
h) it possesses an IR absorption spectrum (in a potassium bromide disk) with spectroscopic absorption bands at: 3290, 2910, 1630, 1530, 1450, 1190, 1150, 1110, 1020, 840, 760, 620, 580 cm⁻¹

i) it possesses a proton NMR absorption spectrum in $D_2O$ with signals at 1.13 (multiplet), 3.36 (multiplet), 4.33 (singlet), 5.1 (singlet), 6.24 (doublet), 8.01 (singlet), 8.81 (singlet), 9.01 (singlet), 9.98 (singlet) ppm;

j) it possesses a $^{13}C$ NMR spectrum in $D_2O$ with signals at:

17.5, 18.25, 18.75, 19.5, 20.02, 21, 23, 25, 25.5, 26.25, 27.5, 27.9, 32, 32.8, 33.56, 36.4, 36.52, 37.5, 42, 42.2, 43, 45.2, 47.2, 49.2, 50.2, 52, 54, 55, 59.5, 60, 60.2, 62.7, 74.7, 65, 67.2, 68, 68.1, 69, 69.9, 70.5, 71.7, 74.2, 75.76, 76.5, 77, 78.2, 81.2, 82.5, 83, 89, 96, 98, 98.7, 100, 102, 103.2, 103.5, 105, 105.2, 105.5, 106.5, 107.5, 109.7, 112, 112.2, 112.5, 112.7, 115, 115.7, 117.5, 118.5, 119.7, 123.5, 127, 128, 128.8, 129, 130, 131.5, 132, 136.9, 137.5, 143, 144.2, 144.7, 144.5, 146.2, 149.5, 150.5, 151, 152.7, 153.5, 153.6, 153.7, 155.5, 156, 157, 158, 160, 164, 163, 166, 167 (readings not taken above 170);

k) the elemental analysis shows the following proportions:

    C: 54-57%

    H: 4.5-5.4%

    O: 36.5-37.5%

    characterised in that:

a) the Mimosa tenuiflora bark is ground or milled or until a ground preparation is obtained,

b) the said ground preparation is treated with a solvent in a proportion of 2:1 to 8:5 W/V, respectively, while heating, and the solvent is separated by filtration, a residue being obtained, which residue is then

c) treated with another solvent in a proportion of 1:3 to 1:12 W/V, respectively, while heating and stirring continuously, and the said solvents are then removed by distillation, a residue being obtained, which residue is dried; it being possible for this step to be repeated several times; and

d) the preparation is subjected to a third extraction with water in a proportion of 1:2 to 1:10 W/V, respectively, while heating and stirring, it being possible for this step to be carried out several times, the aqueous solutions being collected and separated by filtration and being left standing for 12 to 92 hours, and then

e) the aqueous solution is concentrated to 20% of solids by removal of the water, and the product is then placed in an oven until dry, the desired compound being obtained in powder form.

2.    Process according to Claim 1, characterised in that:

    - step b) is carried out with chloroform as solvent in a temperature range between 50 and 70°C;

    - step c) is carried out with ethanol as solvent and in a temperature range between 60 and 80°C;

    - step d) is carried out with water as solvent and in a temperature range between 50 and 100°C.

3.    Process according to Claims 1 and 2, characterised in that the solvents to be used in the extraction are chosen from methanol, acetone, ethyl acetate, dimethyl sulphoxide, hexane, benzene, $CCl_4$, tetrahydrofuran and petroleum ether.

4.    Process according to one of Claims 1 to 3, characterised in that excipients or vehicles are added so as to obtain a pharmaceutical composition.

5.    Process according to Claim 4, characterised in that the quantity of active principle varies between 1 and 70% by weight.

6.    Process according to Claim 4, characterised in that the pharmaceutical composition is in solid or liquid form.

7.    Process according to Claim 4, characterised in that the pharmaceutical composition is in the form of an aqueous solution or of a dusting powder.

FIG. 1

FIG. 2

FIG. 3

FIG. 4